(19) **Europäisches Patentamt / European Patent Office / Office européen des brevets**

(11) **EP 2 932 248 B1**

(12) **EUROPEAN PATENT SPECIFICATION**

(45) Date of publication and mention
of the grant of the patent:
**05.02.2020 Bulletin 2020/06**

(21) Application number: **12813305.5**

(22) Date of filing: **17.12.2012**

(51) Int Cl.:
*G01N 27/02* (2006.01)    *G01N 33/30* (2006.01)
*G01M 13/04* (2019.01)

(86) International application number:
**PCT/EP2012/075707**

(87) International publication number:
**WO 2014/094812 (26.06.2014 Gazette 2014/26)**

(54) **LUBRICATING GREASE CONDITION MONITORING**

ÜBERWACHUNG DES ZUSTANDS VON SCHMIERFETT

SURVEILLANCE DE LA CONDITION D'UNE GRAISSE LUBRIFIANTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**21.10.2015 Bulletin 2015/43**

(73) Proprietor: **Aktiebolaget SKF
415 50 Göteborg (SE)**

(72) Inventors:
• **LUGT, Piet
4132 BB Vianen (NL)**

• **LANG, Defeng
2614 HP Delft (NL)**
• **STORKEN, Jozef Maria
3435 ZN Nieuwegein (NL)**
• **PALLISTER, Dave M.
Highland, Michigan 48357 (US)**

(74) Representative: **Kohl, Thomas et al
SKF GmbH
Gunnar-Wester-Strasse 12
97421 Schweinfurt (DE)**

(56) References cited:
**US-A1- 2011 125 475    US-B1- 7 551 288
US-B2- 7 043 402**

**Description**

BACKGROUND OF THE INVENTION

1. Field of the Invention

The present invention relates to grease condition monitoring and in particular, to use of complex impedance sensing for detecting the level of water or base oil in grease or like substances. The invention also relates to a sensor for monitoring the presence of water and oil in grease and in particular to a method which allows detection of changes to grease condition in-situ.

2. Description of the Related Art

Reliable operation of mechanical systems is highly dependent on correct lubrication. Particularly for systems designed to operate for long periods with little maintenance, the condition of the lubricant is a key factor. The presence of water, particulates or other contaminants can lead to wear and damage of moving parts and even catastrophic failure of the entire mechanical system. Various systems have been implemented for monitoring the condition of oil in circulating lubrication systems. These systems frequently rely on circulation of oil past the sensor and may additionally be provided with filters for removing contaminants.

For non circulating systems, monitoring is more difficult. Closed systems such as bearings are generally filled with an appropriate grease, maintained in position by seals which also protect the grease from ingress of contaminants. Water ingress decreases the lubricating ability of the grease and increases the corrosion damage on the bearing elements. Grease generally comprises a thickener such as a soap or fatty acid, which carries a quantity of lower viscosity base oil that facilitates lubrication. During use of the system, the base oil may gradually separate from the grease and escape from the system e.g. through seals. The remaining grease life can be determined by measuring the remaining oil percentage in the grease.

Capacitive sensors have been proposed for condition monitoring of grease by applying an alternating voltage. Nevertheless, existing systems appear incapable of distinguishing between the presence of water in the grease and a reduction in base oil percentage. It would therefore be desirable to provide for in-situ determination of grease condition, in particular for distinguishing between water ingress and decrease in oil content. It would also be desirable to be able to identify other qualities of the grease, including measurement of a state of oxidation, corrosion damage and the like.

US2011/0125475 discloses a method for monitoring the condition of grease within a rolling element bearing where a voltage between the inner and outer rings of the bearing is applied and a capacitance is measured.

BRIEF SUMMARY OF THE INVENTION

According to the invention there is provided a method of in-situ condition monitoring of grease within a mechanical system being a sealed bearing, the method comprising: providing a pair of electrodes separated by and at least partially in contact with the grease, such that the grease acts as a dielectric between the electrodes; applying a multi-frequency alternating voltage between the electrodes; measuring the resulting current to determine the complex impedance of the equivalent circuit; and comparing the complex impedance with preset values to determine a composition of the grease. As will be understood by the skilled person, the electrodes and the grease together may be represented by an equivalent circuit comprising capacitance and resistance elements. By measuring not only the capacitance but also the resistance of this equivalent circuit, characteristics of the grease can be determined and compared with preset values that may be stored in an appropriate memory. The preset values may be provided in the form of look-up tables, generated in advance for the specific grease contained within the system. It is also conceivable that the preset value may be generated in real time from e.g. a known sample of grease. Evaluating the results at more than one frequency allows specific identification of the grease condition in that it is possible to distinguish between the presence of water and a reduction in base oil. Both of these conditions can lead to deviations in capacitance and resistance but the relative changes are different at different frequencies.

The method may be used to compare the complex impedance with preset values representative of water content of the grease. In this manner an actual water content of the grease may be determined based on a comparison with preset values for different water contents. Alternatively, a single value may be used indicative of an alarm condition and the method may comprise issuing an alarm when the preset value is exceeded.

The method may also be used to compare the complex impedance with preset values representative of oil content of the grease. In this manner, the actual amount of remaining base oil in the grease, or at least an indication thereof, may be determined. Based on these values, a decision may be taken to e.g. stop, service or replace the mechanical system.

The electrodes may be provided in any suitable form, according to the nature of the system. In one embodiment, the electrodes may comprise parallel plates with the grease located therebetween. Such an arrangement may be convenient in the case that grease is supplied to a system, whereby the grease may pass between the plates e.g. forming part of a supply line. In another embodiment, the electrodes comprise adjacent regions on a surface in contact with the grease. The surface could be located on part of a seal or the like. It will be understood that although two electrodes are mentioned, the system could include any number of electrodes or electrode pairs.

Preferably, the alternating voltage is a low voltage source. In particular, the rms voltage may be less than 2 V, preferably less than 1 V and most preferably without DC offset. A voltage applied to the grease may lead to electrolysis or decomposition thereof and can also be detrimental to the electrodes. For this reason, a low voltage is preferred subject to adequate signal strength.

According to a further aspect of the invention, the alternating voltage is preferably applied over a frequency range from 0.1 Hz to 1 MHz, preferably from 1 KHz to 1 MHz. Such a range has been shown as adequate for achieving good signal differentiation using relatively conventional components. It may also be desirable to analyse the signal of specific frequency ranges particular to the oil and to water. In particular, operation at resonant frequencies of these substances may provide greater sensitivity to their detection. The voltage may be applied at a number of discrete frequencies or over a spectrum of frequencies. The complex impedance may be evaluated at individual frequencies or over the full spectrum.

The invention also relates to a mechanical system being a sealed bearing containing lubricating grease and having a condition monitoring sensor comprising: a pair of electrodes separated by and at least partially in contact with the grease, such that the grease acts as a dielectric between the electrodes; a multi-frequency alternating voltage source applied across the electrodes; a signal analyser arranged to analyse the current response to the applied voltages for determining the complex impedance of the equivalent circuit; a memory comprising preset values representative of the expected complex impedance for different conditions of the grease; and a processor for comparing the complex impedance with the preset values in order to determine a composition of the grease. The condition monitoring sensor may operate as described above.

In one preferred embodiment, the system comprises a seal formed of insulating material and at least one of the electrodes is located on the seal. In general, both of the electrodes will be formed on the seal.

According to a further aspect of the invention, the signal analyser comprises a microcircuit in direct electrical contact with the electrodes. The microcircuit may comprise its own source of power. The memory and the processor may form part of the same microcircuit.

According to an alternative embodiment, the processor may be located remotely from the electrodes and the system may comprise a transmission arrangement for transmitting data representative of the current response from the electrodes to the processor.

According to the present invention, the system is a sealed bearing with the sensor provided e.g. on the seal.

BRIEF DESCRIPTION OF THE DRAWINGS

The features and advantages of the invention will be further appreciated upon reference to the following drawings of a number of exemplary embodiments, in which:

Figure 1 shows an equivalent circuit on which measurements according to the invention are based;
Figure 2 shows an experimental setup according to the invention;
Figure 3 shows a graph of water in grease capacitance response as a function of frequency for various water contents;
Figure 4 shows a graph of capacitance measurements for water in grease as a function of water percentages for different frequencies;

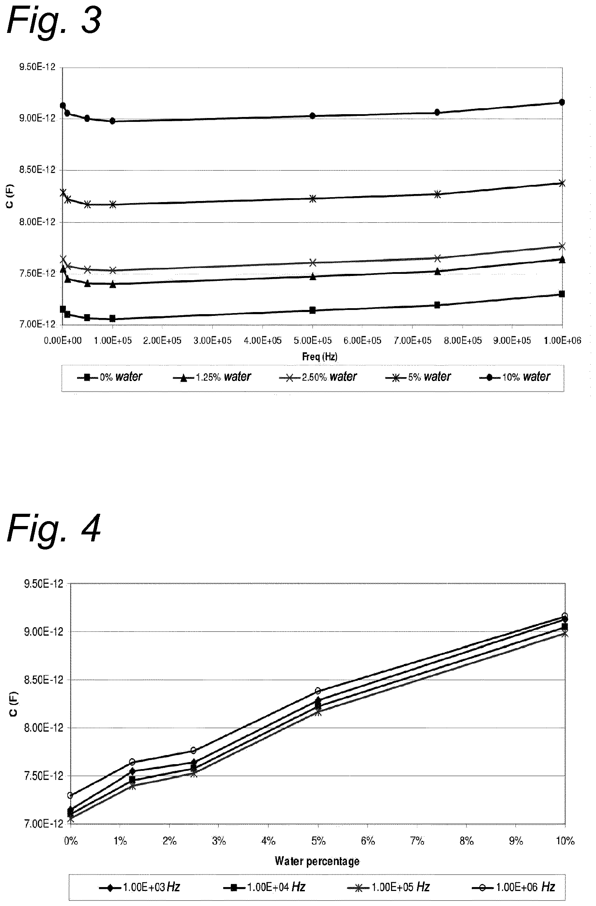

Figure 4 shows a graph of capacitance measurements for water in grease as a function of water percentages for different frequencies;

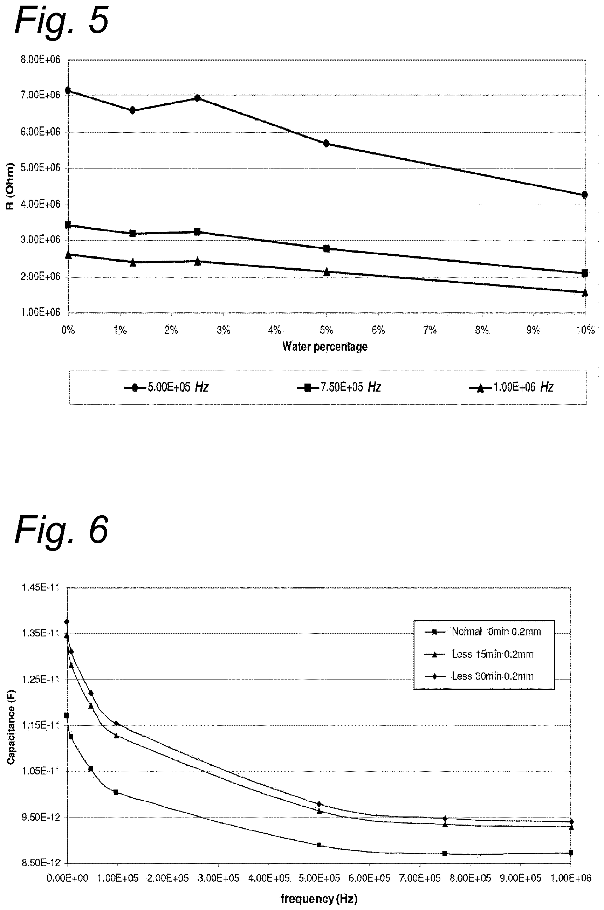

Figure 5 shows a graph of resistance measurements for water in grease as a function of water percentages for different frequencies;
Figure 6 shows a graph of capacitance measurements for oil in grease as a function of frequency for different levels of oil removal;

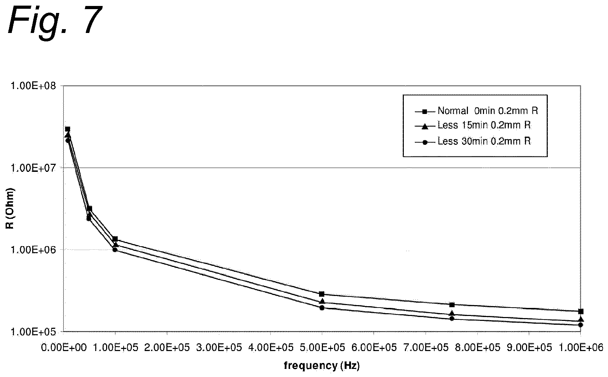

Figure 7 shows a graph of resistance measurements for oil in grease as a function of frequency for different levels of oil removal;

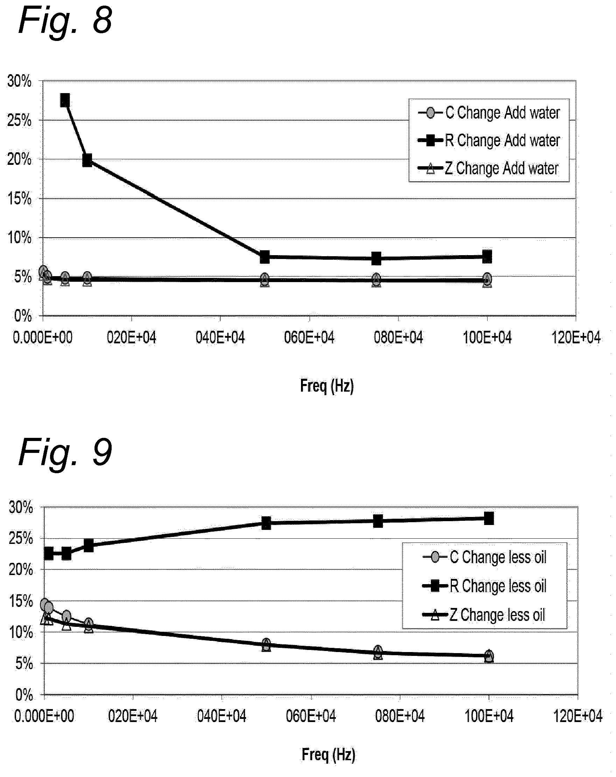

Figure 8 shows the parameter sensitivity of capacitance, resistance and impedance as a function of frequency for the case of 1.25 % water content; and
Figure 9 shows the parameter sensitivity of capacitance, resistance and impedance as a function of frequency for the case of reduced oil content.

DESCRIPTION OF ILLUSTRATIVE EMBODIMENTS

When an electric potential is applied via a pair of electrodes to a material or composition sample, the complex impedance of the sample can be measured. Due to the different electrochemical behaviour of different materials or compositions, unique impedance responses can be identified. By calibrating the spectroscopic impedance pattern for a given grease to the presence of water and loss of base oil, the occurrence of such conditions in sity can be identified.
For a parallel plate capacitor, its capacitance can be described by the dimensions of the plate and the permittivity of the material between the plates, written in Eq. (1).

$$C = \varepsilon_r \varepsilon_0 \frac{A}{d} \tag{1}$$

where

C is the capacitance;
A is the area of overlap of the two plates;
$\varepsilon_r$ is the relative static permittivity of the material between the plates (for a vacuum, $\varepsilon_r$ = 1);
$\varepsilon_0$ is the electric constant ($\varepsilon_0 \sim 8.854 \times 10\text{-}12$ Fm-1); and
d is the separation between the plates.

Lubricating grease consists of thickener and base oil. Both oil and thickener have high resistivities and can be approximated as capacitors. The change of oil percentage will change the overall permittivity of the grease, which results in a different capacity of the system. When water with contaminations, e.g. tap water, is added to grease, the capacitance of the system changes due to the permittivity difference between water and grease. Meanwhile the resistivity of the system drops since tap water causes current leakage in the circuit. The capacitance change due to adding certain percentage (x %) of water can be written as Eq. (2).

$$\begin{aligned} C &= (\varepsilon_{water} \cdot x\% + \varepsilon_{grease} \cdot (1 - x\%) \cdot \varepsilon_0) \frac{A}{d} \\ &= \varepsilon_{water} \varepsilon_0 \cdot x\% \cdot \frac{A}{d} + \varepsilon_{grease} \varepsilon_0 \cdot (1 - x\%) \cdot \frac{A}{d} \\ &= C_{water} + C_{grease} \end{aligned} \tag{2}$$

Where:

C is the capacitance;

$\varepsilon_{water}$ is the relative permittivity of water between the plates ($\varepsilon_{water} \sim 80$);

$\varepsilon_{grease}$ is the relative permittivity of grease between the plates ($\varepsilon_{grease} \sim 2.4$);

$C_{water}$ is the capacitance of water; and

$C_{grease}$ is the capacitance of grease.

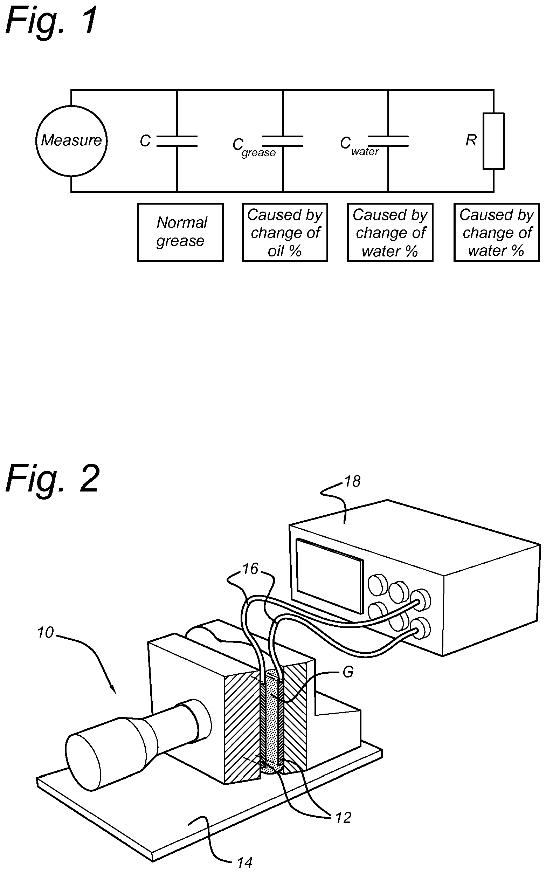

It can be seen from Eq. (2) that when water is added to grease, the initial capacitor C (with only grease) is split into $C_{water}$ and $C_{grease}$, with the $C_{water}$ and $C_{grease}$ connected in parallel. If the change in A or d due to the split of the capacitor are neglected, increasing water in grease will be equivalent to adding a capacitor $C_{water}$ in parallel to $C_{grease}$ in the measurement circuit. Since water with ion contaminations is conductive, a resistance is added to the circuit. The electrical model of this base oil, thickener, water system is shown in Figure 1.

Example 1

An experimental setup 10 of the invention is shown in Figure 2. The experimental setup comprised a parallel plate capacitor construction, in which the separation distance between two metal plates 12 was adjustable. The two plates 12 measured 10 mm x 10 mm each and were mounted in a micrometer 14, directly facing each other. The separation distance could be set from 0 mm to 5 mm with a resolution of 10 micron (0.01 mm) and accuracy of approximately 3 micron. A sample of grease G was placed between the plates 12, ensuring full coverage of both surfaces.

The plates 12 were electrically connected by leads 16 to a programmable automatic RCL meter (sometimes known as LCR meter) 18. The device was a FLUKE PM6306™, equipment number 0501 available from Fluke Corporation. The RCL meter 18 measures the output voltage (V), current (A) and phase difference for a given voltage and frequency input setting. The resistance, impedance and capacitance of the target system can then be calculated. The detailed description of the calculation method is not further described here, being generally conventional and otherwise according to the user manual of the RCL meter.

Samples: Two types of grease samples were used, one for the water in grease test, the other for the oil in grease test. The grease samples used in the water in grease test were SFK GJN, with water percentages of 0%, 1.25%, 2.5%, 5%, and 10% measured in weight. For the oil in grease test, samples of MT33 were used. Samples with different oil content were prepared by putting grease in a centrifuge for 15 minutes and 30 minutes respectively. The experimental setup is shown in Figure 2.

AC voltage input was used for the measurements. In order to reduce electrochemical reaction at the interface between the grease and the plates, the AC input voltage was set to 1 V, which is found sufficiently high for sensing purposes. The DC component of the input was also set to zero to minimize electrolysis on the interface, which otherwise could damage the repeatability for instant measurement or lower stability in a continuous test. Seven frequencies, 1 KHz, 10 KHz, 50 KHz, 100 KHz, 500 KHz, 750 KHz, and 1 MHz, were selected for investigation.

On the RCL meter 18, the circuit selection for the measurement was set to AUTO. At this setting, the device can detect and evaluate whether the target system behaves in a more capacitive or more resistive manner and whether it is more parallel or serially connected. This information is useful for verifying the accuracy of the equivalent circuit. As will be shown later, the water and grease composition indeed appeared as a circuit of parallel capacitors and resistors.

Two separation distances, 0.2 mm and 0.3 mm were used for all tests. For each sample, tests were started with a 0.3 mm gap setting for data acquisition over all 7 frequencies. Next, the test was repeated with the 0.2 mm gap without changing the sample. Before and after testing of each sample the parallel plates were cleaned. The surfaces of the plates were visually inspected for the possible occurrence of corrosion or damage.

The measurement results of the water in grease test with the 0.2 mm gap are shown in Table 1. The phase measurement results showed an angle between -90 to -89 degree. This indicates that the system behaves as a capacitive circuit.

Figure 3 shows the water in grease capacitance response as a function of frequency. The variation in capacitance is less than 3.4%. These results indicate that the capacitance of the composition is not very sensitive to changes in frequency. Figure 4 shows the capacitance measurements for water in grease for different percentages of water at different frequencies. Clearly, the measurements show a consistent result for the various frequencies. The capacitance change is stable and well measurable against the change of water percentage in the grease..

In Figure 5, the resistance versus water percentage in the grease is shown. Similar responses were received when the measurements were done at different frequencies. The electrical resistance decreases with increasing water content. Exceptional results at the 1.25% and 2.5% sample points are found, which are believed to be due to an inaccuracy of the water percentage in the sample or inaccuracy of the separation distance. With the measurement results shown above in Figure 3 to Figure 5, it can be concluded that the assumption of the equivalent electrical circuit shown in Figure 1 is rather reasonable.

Table 1

| Water | Gap (mm) | FREQ (Hz) | V (V) | I (A) | p (degree) | O | C (F) | R (Ohm) | Z (Ohm) |
|---|---|---|---|---|---|---|---|---|---|
| 0% | 0.2 mm | 1.00E+03 | 1 | 4.49E-08 | -89.7 | 207 | 7.15E-12 | 1.00E+12 | 2.23E+07 |
| | 0.2 mm | 1.00E+04 | 1 | 4.46E-07 | -89.8 | 265 | 7.10E-12 | 1.00E+12 | 2.24E+06 |
| | 0.2 mm | 5.00E+04 | 1 | 2.22E-06 | -89.8 | 278 | 7.07E-12 | 1.25E+08 | 4.50E+05 |
| | 0.2 mm | 1.00E+05 | 1 | 4.44E-06 | -89.8 | 313 | 7.06E-12 | 7.05E+07 | 2.25E+05 |
| | 0.2 mm | 5.00E+05 | 1 | 2.24E-05 | -89.6 | 160 | 7.14E-12 | 7.14E+06 | 4.46E+04 |
| | 0.2 mm | 7.50E+05 | 1 | 3.39E-05 | -89.5 | 116.2 | 7.19E-12 | 3.43E+06 | 2.95E+04 |

(continued)

| Water | Gap (mm) | FREQ (Hz) | V (V) | I (A) | p (degree) | O | C (F) | R (Ohm) | Z (Ohm) |
|---|---|---|---|---|---|---|---|---|---|
|  | 0.2 mm | 1.00E+06 | 1 | 4.59E-05 | -89.5 | 119.3 | 7.30E-12 | 2.60E+06 | 2.18E+04 |
| 1.25% | 0.2 mm | 1.00E+03 | 1 | 4.74E-08 | -89.2 | 72.3 | 7.55E-12 | 1.00E+12 | 2.11E+07 |
|  | 0.2 mm | 1.00E+04 | 1 | 4.68E-07 | -89.6 | 149.2 | 7.45E-12 | 1.00E+12 | 2.14E+06 |
|  | 0.2 mm | 5.00E+04 | 1 | 2.33E-06 | -89.7 | 211 | 7.41E-12 | 9.06E+07 | 4.30E+05 |
|  | 0.2 mm | 1.00E+05 | 1 | 4.65E-06 | -89.8 | 263 | 7.40E-12 | 5.65E+07 | 2.15E+05 |
|  | 0.2 mm | 5.00E+05 | 1 | 2.35E-05 | -89.6 | 155 | 7.47E-12 | 6.60E+06 | 4.26E+04 |
|  | 0.2 mm | 7.50E+05 | 1 | 3.54E-05 | -89.5 | 112.8 | 7.52E-12 | 3.18E+06 | 2.82E+04 |
|  | 0.2 mm | 1.00E+06 | 1 | 4.80E-05 | -89.5 | 115.4 | 7.64E-12 | 2.41E+06 | 2.08E+04 |
| 2.50% | 0.2 mm | 1.00E+03 | 1 | 4.80E-08 | -89.6 | 127.3 | 7.64E-12 | 1.00E+12 | 2.08E+07 |
|  | 0.2 mm | 1.00E+04 | 1 | 4.76E-07 | -89.8 | 235 | 7.57E-12 | 1.00E+12 | 2.10E+06 |
|  | 0.2 mm | 5.00E+04 | 1 | 2.37E-06 | -89.8 | 275 | 7.54 E-12 | 1.16E+08 | 4.22E+05 |
|  | 0.2 mm | 1.00E+05 | 1 | 4.73E-06 | -89.8 | 332 | 7.53E-12 | 7.01E+07 | 2.11E+05 |
|  | 0.2 mm | 5.00E+05 | 1 | 2.39E-05 | -89.7 | 165.4 | 7.61E-12 | 6.92E+06 | 4.18E+04 |
|  | 0.2 mm | 7.50E+05 | 1 | 3.61E-05 | -89.5 | 117 | 7.65E-12 | 3.24E+06 | 2.77E+04 |
|  | 0.2 mm | 1.00E+06 | 1 | 4.88E-05 | -89.5 | 118.2 | 7.76E-12 | 2.42E+06 | 2.05E+04 |
| 5% | 0.2 mm | 1.00E+03 | 1 | 5.21E-08 | -89.4 | 100.8 | 8.29E-12 | 1.00E+12 | 1.92E+07 |
|  | 0.2 mm | 1.00E+04 | 1 | 5.16E-07 | -89.7 | 194.5 | 8.22E-12 | 1.00E+12 | 1.94E+06 |
|  | 0.2 mm | 5.00E+04 | 1 | 2.57E-06 | -89.8 | 234 | 8.17E-12 | 9.10E+07 | 3.89E+05 |
|  | 0.2 mm | 1.00E+05 | 1 | 5.13E-06 | -89.8 | 274 | 8.17E-12 | 5.33E+07 | 1.95E+05 |
|  | 0.2 mm | 5.00E+05 | 1 | 2.59E-05 | -89.6 | 146.8 | 8.23E-12 | 5.68E+06 | 3.87E+04 |
|  | 0.2 mm | 7.50E+05 | 1 | 3.90E-05 | -89.5 | 108.4 | 8.27E-12 | 2.78E+06 | 2.57E+04 |
|  | 0.2 mm | 1.00E+06 | 1 | 5.27E-05 | -89.5 | 112.9 | 8.38E-12 | 2.14E+06 | 1.90E+04 |
| 10% | 0.2 mm | 1.00E+03 | 1 | 5.74E-08 | -89.5 | 117.4 | 9.13E-12 | 1.00E+12 | 1.74E+07 |
|  | 0.2 mm | 1.00E+04 | 1 | 5.68E-07 | -89.7 | 183.5 | 9.05E-12 | 1.00E+12 | 1.76E+06 |
|  | 0.2 mm | 5.00E+04 | 1 | 2.83E-06 | -89.7 | 208 | 9.00E-12 | 7.35E+07 | 3.54E+05 |
|  | 0.2 mm | 1.00E+05 | 1 | 5.64E-06 | -89.8 | 230 | 8.98E-12 | 4.08E+07 | 1.77E+05 |

(continued)

| Water | Gap (mm) | FREQ (Hz) | V (V) | I (A) | p (degree) | O | C (F) | R (Ohm) | Z (Ohm) |
|---|---|---|---|---|---|---|---|---|---|
| | 0.2 mm | 5.00E+05 | 1 | 2.84E-05 | -89.5 | 121 | 9.03E-12 | 4.26E+06 | 3.52E+04 |
| | 0.2 mm | 7.50E+05 | 1 | 4.27E-05 | -89.4 | 89.6 | 9.06E-12 | 2.10E+06 | 2.34E+04 |
| | 0.2 mm | 1.00E+06 | 1 | 5.76E-05 | -89.4 | 90.6 | 9.16E-12 | 1.57E+06 | 1.74E+04 |

Example 2

The same test method and procedures were adopted for measuring the remaining oil in grease. In this example grease samples of MT33 grease were used. A first sample was fresh grease. Second and third samples with reduced oil content were prepared by centrifuging the grease for 15 minutes and 30 minutes respectively.

The capacitance response chart of the three grease samples over seven frequencies is shown in Figure 6. The separation distance of the setup was 0.2 mm. The measurements show a general trend of capacitance increase against lessening of oil. Similar results were recorded with a 0.3 mm gap between the plates. The measurements show some spread. This is believed to be due to the nature of the centrifuging process for removing the oil, which causes it to be unevenly distributed within the grease. Two portions of the same sample may thus actually contain different amounts of oil.

The resistance response of the three grease samples over the frequency range is shown in Figure 7. A clear resistance difference can be seen between the fresh grease and the grease with reduced oil content. The grease that contains less oil has a somewhat lower electrical resistance than the fresh grease. However the differences are not very pronounced.

For further implementation of the technique into a grease sensor, the sensitivity of the various parameters to changes in grease composition is favoured. The parameter sensitivity for capacitance is defined as:

$$Sensitivity = \frac{C_{1.25\%} - C_{0\%}}{C_{0\%}}$$

Similar sensitivities are calculated for resistance and impedance. Figure 8 shows the parameter sensitivity of capacitance, resistance and impedance over the frequency range for the case of 1.25 % water content.

Figure 9 shows the same parameter sensitivities for the case of MT33 grease with respect to the same grease with a reduced oil content based on 15 minutes centrifuging. On comparing Figures 8 and 9, it can be noted that the parameter sensitivities due to changes in water content are significantly different to those due to reduction in oil content. In particular, the slope of the graphs is distinct, and can be used to identify and distinguish a change of water content from a change of oil content.

**Claims**

1.    A method of in-situ condition monitoring of grease within a mechanical system being a sealed bearing, the method being **characterised in that** it comprises:

providing a pair of electrodes separated by and at least partially in contact with the grease, such that the grease acts as a dielectric between the electrodes;
applying a multi-frequency alternating voltage between the electrodes;
measuring the resulting current to determine a complex impedance of an equivalent circuit;
comparing the complex impedance with preset values to determine a composition of the grease.

2.    The method according to claim 1, wherein the electrodes comprise parallel plates with the grease located therebetween.

3.    The method according to claim 1, wherein the electrodes comprise adjacent regions on a surface in contact with the grease.

4. The method according to any preceding claim, wherein the alternating voltage is less than 2 V, preferably less than 1 V.

5. The method according to any preceding claim, wherein the alternating voltage is applied over a frequency range from 0.1 Hz to 1 MHz, preferably from 1 KHz to 1 MHz.

6. The method according to any preceding claim, wherein the alternating voltage is applied over a spectrum of frequencies and the complex impedance is evaluated over the spectrum.

7. The method according to any preceding claim, comprising comparing the complex impedance with preset values representative of water content of the grease and determining the actual water content of the grease based on the comparison.

8. The method according to any preceding claim, comprising comparing the complex impedance with preset values representative of oil content of the grease and determining the actual oil content of the grease based on the comparison.

9. A mechanical system being a sealed bearing containing lubricating grease and having a condition monitoring sensor, said system being **characterised in that** it comprises:

   a pair of electrodes separated by and at least partially in contact with the grease, such that the grease acts as a dielectric between the electrodes;
   a multi-frequency alternating voltage source applied across the electrodes;
   a signal analyser arranged to analyse the current response to the applied voltages for determining a complex impedance of an equivalent circuit;
   a memory comprising preset values representative of the expected complex impedance for different conditions of the grease; and
   a processor for comparing the complex impedance with the preset values in order to determine a composition of the grease.

10. The system according to claim 9, wherein the electrodes comprise parallel plates with the grease located therebetween.

11. The system according to claim 9, wherein the electrodes comprise adjacent regions on a surface in contact with the grease.

12. The system according to any of claims 9 to 11, wherein the system comprises a seal formed of insulating material and at least one of the electrodes is located on the seal.

13. The system according to any of claims 9 to 12, wherein the signal analyser comprises a microcircuit in electrical contact with the electrodes.

14. The system according to any of claims 9 to 13, wherein the processor is located remotely from the electrodes and the system comprises a transmission arrangement for transmitting data representative of the current response from the electrodes to the processor.

15. The system according to any of claims 9 to 14, wherein the system is a sealed bearing.


**Patentansprüche**

1. Verfahren einer In-Situ-Überwachung eines Zustands von Schmiermittel in einem mechanischen System, das ein abgedichtetes Lager ist, **dadurch gekennzeichnet, dass** es umfasst:

   Bereitstellen eines Paars von Elektroden, die getrennt sind durch und zumindest teilweise in Kontakt sind mit dem Schmiermittel, sodass das Schmiermittel als ein Dielektrikum zwischen den Elektroden wirkt;
   Anlegen einer Mehrfrequenzwechselspannung zwischen den Elektroden;
   Messen des resultierenden Stroms, um eine komplexe Impedanz einer Äquivalenzschaltung zu bestimmen;
   Vergleichen der komplexen Impedanz mit vorbestimmten Werten, um eine Zusammensetzung des Schmiermittels zu bestimmen.

2. Verfahren nach Anspruch 1, wobei die Elektroden parallele Platten umfassen, zwischen denen sich das Schmiermittel befindet.

3. Verfahren nach Anspruch 1, wobei die Elektroden benachbarte Regionen an einer Fläche in Kontakt mit dem Schmiermittel umfassen.

4. Verfahren nach einem der vorhergehenden Ansprüche, wobei die Wechselspannung kleiner als 2 V, vorzugsweise kleiner als 1 V ist.

5. Verfahren nach einem der vorhergehenden Ansprüche, wobei die Wechselspannung über einen Frequenzbereich von 0,1 Hz bis 1 MHz, vorzugsweise von 1 KHz bis 1 MHz angelegt wird.

6. Verfahren nach einem der vorhergehenden Ansprüche, wobei die Wechselspannung über ein Spektrum von Frequenzen angelegt wird und die komplexe Impedanz über das Spektrum ausgewertet wird.

7. Verfahren nach einem der vorhergehenden Ansprüche, umfassend Vergleichen der komplexen Impedanz mit vorbestimmten Werten, die einen Wassergehalt des Schmiermittels anzeigen, und Bestimmen des tatsächlichen Wassergehalts des Schmiermittels anhand des Vergleichs.

8. Verfahren nach einem der vorhergehenden Ansprüche, umfassend Vergleichen der komplexen Impedanz mit vorbestimmten Werten, die einen Ölgehalt des Schmiermittels anzeigen, und Bestimmen des tatsächlichen Ölgehalts des Schmiermittels anhand des Vergleichs.

9. Mechanisches System, das ein abgedichtetes Lager ist, das Schmiermittel enthält und das einen Zustandsüberwachungssensor aufweist, wobei das System **dadurch gekennzeichnet ist, dass** es umfasst:

ein Paar von Elektroden, die getrennt sind durch und zumindest teilweise in Kontakt sind mit dem Schmiermittel, sodass das Schmiermittel als ein Dielektrikum zwischen den Elektroden wirkt;
eine Mehrfrequenzwechselspannungsquelle, die über die Elektroden angelegt ist;
einen Signalanalysator, der ausgestaltet ist, eine Stromantwort auf die angelegten Spannungen zu analysieren, um eine komplexe Impedanz einer Äquivalenzschaltung zu bestimmen;
einen Speicher, umfassend vorbestimmte Werte, die eine erwartete komplexe Impedanz für unterschiedliche Zustände des Schmiermittels anzeigen; und
einen Prozessor zum Vergleichen der komplexen Impedanz mit den vorbestimmten Werten, um eine Zusammensetzung des Schmiermittels zu bestimmen.

10. System nach Anspruch 9, wobei die Elektroden parallele Platten umfassen, zwischen denen sich das Schmiermittel befindet.

11. System nach Anspruch 9, wobei die Elektroden benachbarte Regionen an einer Fläche in Kontakt mit dem Schmiermittel umfassen.

12. System nach einem der Ansprüche 9 bis 11, wobei das System eine Dichtung umfasst, die aus Isolationsmaterial ausgebildet ist, und sich mindestens eine der Elektroden an der Dichtung befindet.

13. System nach einem der Ansprüche 9 bis 12, wobei der Signalanalysator eine Mikroschaltung in elektrischem Kontakt mit den Elektroden umfasst.

14. System nach einem der Ansprüche 9 bis 13, wobei sich der Prozessor entfernt von den Elektroden befindet und das System eine Übertragungsanordnung zum Übertragen von Daten umfasst, die die Stromantwort von den Elektroden zu dem Prozessor anzeigen.

15. System nach einem der Ansprüche 9 bis 14, wobei das System ein abgedichtetes Lager ist.

**Revendications**

1. Procédé de surveillance in situ de l'état d'une graisse au sein d'un système mécanique consistant en un roulement étanche, le procédé étant **caractérisé en ce qu'**il comprend :

   la fourniture d'une paire d'électrodes séparées par la graisse et au moins partiellement à son contact, de sorte que la graisse joue le rôle d'un diélectrique entre les électrodes ;
   l'application d'une tension alternative multi-fréquence entre les électrodes ;
   la mesure du courant résultant afin de déterminer une impédance complexe d'un circuit équivalent ;
   la comparaison de l'impédance complexe à des valeurs préétablies afin de déterminer une composition de la graisse.

2. Procédé selon la revendication 1, dans lequel les électrodes comprennent des plaques parallèles entre lesquelles se situe la graisse.

3. Procédé selon la revendication 1, dans lequel les électrodes comprennent des régions adjacentes sur une surface au contact de la graisse.

4. Procédé selon l'une quelconque des revendications précédentes, dans lequel la tension alternative est inférieure à 2 V, de préférence inférieure à 1 V.

5. Procédé selon l'une quelconque des revendications précédentes, dans lequel la tension alternative est appliquée sur l'étendue d'une plage de fréquences allant de 0,1 Hz à 1 MHz, de préférence de 1 kHz à 1 MHz.

6. Procédé selon l'une quelconque des revendications précédentes, dans lequel la tension alternative est appliquée sur l'étendue d'un spectre de fréquences et l'impédance complexe est évaluée sur l'étendue du spectre.

7. Procédé selon l'une quelconque des revendications précédentes, comprenant la comparaison de l'impédance complexe à des valeurs préétablies représentant une teneur en eau de la graisse et la détermination de la teneur réelle en eau de la graisse sur la base de la comparaison.

8. Procédé selon l'une quelconque des revendications précédentes, comprenant la comparaison de l'impédance complexe à des valeurs préétablies représentant une teneur en huile de la graisse et la détermination de la teneur réelle en huile de la graisse sur la base de la comparaison.

9. Système mécanique consistant en un roulement étanche contenant de la graisse lubrifiante et doté d'un capteur de surveillance d'état, ledit système étant **caractérisé en ce qu'**il comprend :

   une paire d'électrodes séparées par la graisse et au moins partiellement à son contact, de sorte que la graisse joue le rôle d'un diélectrique entre les électrodes ;
   une source de tension alternative multi-fréquence appliquée aux bornes des électrodes ;
   un analyseur de signal conçu pour analyser la réponse en courant aux tensions appliquées aux fins de déterminer une impédance complexe d'un circuit équivalent ;
   une mémoire comprenant des valeurs préétablies représentant l'impédance complexe attendue pour différents états de la graisse ; et
   un processeur destiné à comparer l'impédance complexe aux valeurs préétablies dans le but de déterminer une composition de la graisse.

10. Système selon la revendication 9, dans lequel les électrodes comprennent des plaques parallèles entre lesquelles se situe la graisse.

11. Système selon la revendication 9, dans lequel les électrodes comprennent des régions adjacentes sur une surface au contact de la graisse.

12. Système selon l'une quelconque des revendications 9 à 11, lequel système comprend un joint d'étanchéité formé de matériau isolant et au moins une des électrodes se situe sur le joint d'étanchéité.

13. Système selon l'une quelconque des revendications 9 à 12, dans lequel l'analyseur de signal comprend un microcircuit au contact électrique des électrodes.

14. Système selon l'une quelconque des revendications 9 à 13, dans lequel le processeur se situe à distance des électrodes, et lequel système comprend un agencement de transmission destiné à transmettre des données représentant la réponse en courant depuis les électrodes vers le processeur.

15. Système selon l'une quelconque des revendications 9 à 14, lequel système consiste en un roulement étanche.

*Fig. 1*

| Normal grease | Caused by change of oil % | Caused by change of water % | Caused by change of water % |

*Fig. 2*

## Fig. 3

## Fig. 4

## Fig. 5

## Fig. 6

# Fig. 7

## Fig. 8

Freq (Hz)

## Fig. 9

Freq (Hz)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20110125475 A